(19) Europäisches Patentamt
European Patent Office
Office européen des brevets

(11) Publication number: **0 657 535 A2**

(12) **EUROPEAN PATENT APPLICATION**

(21) Application number: **94117847.7**

(22) Date of filing: **11.11.94**

(51) Int. Cl.[6]: **C12N 15/55**, C12N 9/20, C12N 15/67, C12P 21/02, //C12R1:38

(30) Priority: **19.11.93 JP 314314/93**

(43) Date of publication of application: **14.06.95 Bulletin 95/24**

(84) Designated Contracting States: **DE DK GB**

(71) Applicant: **CHISSO CORPORATION**
**6-32, Nakanoshima 3-chome**
**Kitaku**
**Osaka (JP)**

(72) Inventor: **Aoyama, Shigeyuki**
**10-2, Otsutomocho,**
**Kanazawaku**
**Yokohamashi,**
**Kanagawaken (JP)**
Inventor: **Yoshida, Naoyuki**
**1750, Murakami**
**Ichiharashi,**
**Chibaken (JP)**

(74) Representative: **Kraus, Walter, Dr. et al**
**Patentanwälte Kraus, Weisert & Partner**
**Thomas-Wimmer-Ring 15**
**D-80539 München (DE)**

(54) **A lipase gene originated from pseudomonas bacteria.**

(57) A lipase originated from Pseudomonas genus bacterium, having a commercially high utilization value, and a process for producing the lipase are provided,

which lipase is a recombinant DNA containing a lipase gene originated from Pseudomonas cepacia IFO 14595 strain having a DNA sequence of the following sequence No. 1, and a gene needed for its activity expression:

EP 0 657 535 A2

Sequence No. 1

```
ATGGCCAGAT CGATGCGTTC CAGGGTGGTG GCAGGGGCAG TGGCATGCGC GATGAGCGTC   60
GCGCCGTTCG CGGGGACGAC CGCAGTGATG ACGCTTGCGA CGACGCACGC AGCGATGGCG  120
GCGACCGCGC CCGCCGACGA CTACGCGACG ACGCGTTATC CGATCATCCT CGTGCACGGG  180
CTCACGGGCA CCGACAAGTA CGCGGGCGTG CTCGAGTACT GGTACGGCAT CCAGGAAGAC  240
CTGCAGCAGC ATGGCGCGAC CGTCTACGTC GCGAACCTGT CGGGCTTCCA GAGCGATGAC  300
GGCCCGAACG GGCGCGGCGA ACAGCTGCTC GCTTACGTGA AGACGGTGCT CGCCGCGACG  360
GGCGCGACCA AGGTCAATCT CGTCGGTCAC AGCCAGGGCG GGCTCACGTC GCGTTATGTC  420
GCGGCCGTCG CGCCCGATCT CGTCGCGTCG GTGACGACGA TCGGCACGCC GCATCGCGGC  480
TCCGAGTTCG CCGACTTCGT GCAGAGCGTG CTCGCATACG ATCCGACCGG GCTTTCGTCG  540
TCGGTGATCG CCGCGTTCGT CAATGTGTTC GGAATCCTGA CGAGCAGCAG TCACAACACG  600
AACCAGGACG CGCTCGCGTC GCTGAAGACG CTGACGACCG CACAGGCCGC CACGTACAAC  660
CAGAACTATC CGAGCGCGGG CCTTGGCGCG CCGGGCAGTT GCCAGACCGG CGCACCGACG  720
GAAACCGTCG GCGGCAACAC GCATCTGCTG TATTCGTGGG CCGGCACGGC GATCCAGCCG  780
ACGCTCTCCG TGTTCGGTGT CACGGGTGCG ACGGACACGA GCACCATTCC GCTCGTCGAC  840
CCGGCGAACG CGCTCGATCT GTCGACGCTC GCGCTGTTCG GCACGGGCAC GGTGATGATC  900
AACCGCGGCT CGGGCCAGAA CGACGGGCTC GTGTCGAAGT GCAGCGCGCT GTACGGCCAG  960
GTGCTGAGCA CGAGCTACAA GTGGAACCAT ATCGACGAGA TCAACCAGTT GCTTGGCGTG 1020
CGCGGCGCGT ATGCGGAAGA TCCGGTCGCG GTGATCCGCA CGCATGCGAA CCGGCTGAAA 1080
CTGGCGGGCG TGTAA                                                 1095
```

and which process comprises carrying out secretory production of lipase into the cultured filtrate using the recombinant DNA,

according to which process, it is possible to recover the lipase from the cultured filtrate at the time of commercial production; hence it is possible to supply a cheap lipase.

BACKGROUND OF THE INVENTION

1. Field of the Invention

This invention relates to a DNA sequence of a gene coding a lipase originated from Pseudomonas bacteria, a recombinant DNA containing the DNA sequence, and a process for cultivating a host species bacterium transformed by the recombinant DNA and secretorily producing a lipase into the cultured filtrate.

2. Description of the Related Art

Lipase has been used as an enzyme for hydrolyzing lipids, for oil and fat processing, clinical dignostic, detergent, digestant, etc., and besides, in recent years, it is an important enzyme for catalyzing ester hydrolysis, ester synthesis, or ester interchange, as a process for producing chemical products, particularly optically active compounds.

Further, Pseudomonas genus bacteria have been known to produce a lipase useful as a catalyst for ester hydrolysis, ester synthesis or ester interchange (see Japanese patent application laid-open No. Sho 62-166898). Further, these lipases have been utilized industrially in a large quantity. Thus, lipases for industrial enzymes have been desired to be supplied in a cheap cost, besides heat stability and characteristic substrate-specificity.

In recent years, due to development of recombinant DNA technique, genes coding enzemes which have industrial utility value have been isolated, and their DNA sequences have been clarified. Further, making use of the technique, it has been studied or carried out to produce enzymes having improved the production efficiency and having improved the stability to heat, organic solvents, etc. or those characteristic of substrate-specificity. As to lipase originated from Pseudomonas genus bacteria, too, the genes thereof have been isolated and the primary structure thereof and a process for producing recombinant lipases have been reported (see Japanese patent application laid-open Nos. Sho 63-187684, Sho 64-74992, Hei 2-190188 and Hei 3-47079).

As to these lipases, Escherichia coli advantageous to produce recombinant proteins has been used as a host species bacterium because various factors for producing the lipases have been known and a number of expression Proteins therefor, etc. have been developed. However, such conventional processes have been characterized in that recombinant lipases have been produced by crushing Escherichia coli by means of supersonic wave treatment, etc.; hence such processes are disadvantageous in the aspect of commercial production. Accordingly, it has been required to develop a host species bacterium having DNA characterized by secretorily produce a lipase into the cultured filtrate, which is advantageous in the aspect of commercial production.

In general, as to secretory production of different kinds of proteins, there are a number of unknown matters, and it is necessary to investigate various factors such as the primary structure of protein to be secretorily produced, host species bacterium, signal peptide, etc. Thus, in the case of the lipase of Pseudomonas species bacteria, too, these factors and genes adjusting the expression of lipase activity should be investigated (see Japanese patent application laid-open No. Hei 3-87187 and Hei 4-169184).

Problems to be Solved by the Invention

In such a situation, the present inventors have aimed to derive a lipase originated from Pseudomonas genus bacterium into a product having a higher utility value in the aspect of heat stability, substrate-specificity, etc. to thereby utilize it commercially in a large quantity, and thereby firstly develop a host species bacterium having a DNA characterized by producing a lipase in the cultured filtrate which is advantageous in the aspect of commercial production, in a good efficiency and in a large quantity. As a result of extensive research, the present inventors have carried out cloning of a lipase gene of Pseudomonas cepacia IFO 14595 strain and determination of its DNA sequence, and have constructed a system of a host species bacterium-a recombinant DNA for producing a lipase, characterized by producing a lipase in the cultured filtrate, using the host genus bacterium having introduced therein the recombinant DNA containing the above DNA sequence, and have completed the present invention.

As apparent from the foregoing, the object of the present invention is to derive a lipase which is commercially highly useful, and to provide an advantageous productivity thereof.

## SUMMARY OF THE INVENTION

The present invention has the following constitutions (1) to (7):

(1) A lipase gene originated from Pseudomonas genus bacterium, characterized by having a DNA sequence of sequence No. 1:

Length of sequence: 1095
Type of sequence: nucleic acid
Number of chains: double-stranded
Topology: linear
Kind of sequence: Genomic DNA
Origin:
Name of organism: Pseudomonas cepacia
Name of strain: IFO 14595
Sequence:

```
ATGGCCAGAT CGATGCGTTC CAGGGTGGTG GCAGGGGCAG TGGCATGCGC GATGAGCGTC   60
GCGCCGTTCG CGGGGACGAC CGCAGTGATG ACGCTTGCGA CGACGCACGC AGCGATGGCG  120
GCGACCGCGC CCGCCGACGA CTACGCGACG ACGCGTTATC CGATCATCCT CGTGCACGGG  180
CTCACGGGCA CCGACAAGTA CGCGGGCGTG CTCGAGTACT GGTACGGCAT CCAGGAAGAC  240
CTGCAGCAGC ATGGCGCGAC CGTCTACGTC GCGAACCTGT CGGGCTTCCA GAGCGATGAC  300
GGCCCGAACG GGCGCGGCGA ACAGCTGCTC GCTTACGTGA AGACGGTGCT CGCCGCGACG  360
GGCGCGACCA AGGTCAATCT CGTCGGTCAC AGCCAGGGCG GGCTCACGTC GCGTTATGTC  420
GCGGCCGTCG CGCCCGATCT CGTCGCGTCG GTGACGACGA TCGGCACGCC GCATCGCGGC  480
TCCGAGTTCG CCGACTTCGT GCAGAGCGTG CTCGCATACG ATCCGACCGG GCTTTCGTCG  540
TCGGTGATCG CCGCGTTCGT CAATGTGTTC GGAATCCTGA CGAGCAGCAG TCACAACACG  600
AACCAGGACG CGCTCGCGTC GCTGAAGACG CTGACGACCG CACAGGCCGC CACGTACAAC  660
CAGAACTATC CGAGCGCGGG CCTTGGCGCG CCGGGCAGTT GCCAGACCGG CGCACCGACG  720
GAAACCGTCG GCGGCAACAC GCATCTGCTG TATTCGTGGG CCGGCACGGC GATCCAGCCG  780
ACGCTCTCCG TGTTCGGTGT CACGGGTGCG ACGGACACGA GCACCATTCC GCTCGTCGAC  840
CCGGCGAACG CGCTCGATCT GTCGACGCTC GCGCTGTTCG GCACGGGCAC GGTGATGATC  900
AACCGCGGCT CGGGCCAGAA CGACGGGCTC GTGTCGAAGT GCAGCGCGCT GTACGGCCAG  960
GTGCTGAGCA CGAGCTACAA GTGGAACCAT ATCGACGAGA TCAACCAGTT GCTTGGCGTG 1020
CGCGGCGCGT ATGCGGAAGA TCCGGTCGCG GTGATCCGCA CGCATGCGAA CCGGCTGAAA 1080
CTGGCGGGCG TGTAA                                                 1095
```

(2) A gene coding a factor necessary for expressing the lipase activity originated from Pseudomonas genus bacterium, characterized by having a DNA sequence of sequence No. 2:

Length of sequence: 1035
Type of sequence: nucleic acid
Number of chains: double-stranded

Topology: linear
Kind of sequence: Genomic DNA
Origin:
Name of organism: Pseudomonas cepacia
Name of strain: IFO 14595
Sequence:

```
ATGACGGCAC GTGAAGGGCG CGCGCCGCGG GCGCGGCGCG CTGTGGTCTA CGGTGTCGCG   60
GGGCTGGCGG CGATCGTCGG CGTCGCGATG TGGAGCGGTG CGGGATGGCA TCGCGATACG  120
GGTAGCACCG GCGAGTCGCC CGATGCTGCG GCGGTGGGCG GCGTGGCTGC GGCACCGCCG  180
CAGGCCGCCG TGCCGGCGAG CGCGGGCCTG CCGTCGTCGC TGGCCGGTTC CAGCGCGCCG  240
CGGCTGCCGC TCGATGCCGG CGGCCATCTC GCGAAGGTGC GCGCGGTACG CGATTTCTTC  300
GATTACTGCC TGACCGCGCA GAGCGATCTC AGTACGGCCG CGCTCGATGC GCTCGTCGCG  360
CGCGAGATTG CCGCGCAGCT CGACGGTACG GTTGCGCAGG CCGACGCGCT CGACGTGTGG  420
CGCCGGTATC GCGCGTATCT CGACGCGCTC GCGAAACTGC GCGATGCCGG CGCGGTCGAC  480
AAGTCCGACC TGGGCGCGCT GCAGCTCGCG CTCGACCAGC GCGCGTCGAT CGCGTATCGC  540
ACGCTCGGCG ACTGGAGTCA GCCGTTCTTC GGCGCGGAGC AGTGGCGGCA GCGCTACGAT  600
CTCGCACGGC TGAAGATCGC GCAGGATCGC ACGCTGACCG ATGCGCAGAA GGCCGAACGG  660
CTCGCGGCGC TCGAGCAGCA GATGCCGGCC GACGAACGCG CGGCGCACGA GCGGGTCGAC  720
CGGCAGCGGG CCGCGATCGA CCAGATCGCG CAGTTGCAGA AGAGCGGGGC GACGCCCGAT  780
GCGATGCGCG CGCAACTGAC GCAGACGCTC GGGCCCGAGG CCGCCGCACG CGTCGCGCAG  840
ATGCAGCAGG ACGACGCATC GTGGCAGAGC CGCTACGCGG ACTATGCGGC ACAGCGTGCG  900
CAGATCGAGT CGGCCGGCCT GTCGCCGCCG GACCGCGACG CGCAGATCGC CGCACTGCGG  960
CAGCGCATGT TCACGAAGCC CGGCGAAGCC GTGCGCGCGG CGTCGCTCGA TCGCGGCGCG 1020
GGCAGCGCGC AGTAA                                                 1035
```

(3) A lipase gene according to item (1), wherein said Pseudomonas genus bacterium is Pseudomonas cepacia IFO 14595 strain.

(4) A recombinant DNA having incorporated therein, a lipase gene or a gene coding a factor necessary for expressing a lipase gene according to either one of items (1), (2) or (3).

(5) A host species bacterium transformed by said recombinant DNA according to item (4).

(6) A process for producing a lipase, which comprises cultivating said host species bacterium according to item (5) and secretorily producing a lipase in the cultured filtrate.

(7) A process for producing a lipase according to item (6) wherein the vector according to item (4) is a vector for Eschericia coli and the host genus bacterium according to item (5) is Escherichia coli.

BRIEF DESCRIPTION OF THE ACCOMPANYING DRAWINGS

Fig. 1 shows an example of a restriction enzyme map of pCPC-inserted Eco RI 13.5 kb DNA fragment, and results of a defect experiment employing as an indication, the presence or absence of clear zone formation at the time of subcloning into a plasmid pUC 119. " + " refers to clear zone formation.

Fig. 2 shows the amino acid sequence of the gene of the present invention.

Fig. 3 shows the amino acid sequence succeeding to that of Fig. 2.

Fig. 4 shows the amino acid sequence succeeding to that of Fig. 3.

Fig. 5 shows plasmid of lipase-secretory-production for Escherichia coli, pTV-CPC mut N.

DETAILED DESCRIPTION OF PREFERRED EMBODIMENTS

The present invention will be described in more detail.

(1) Cloning of lipase gene

Pseudomonas cepacia IFO 14595 strain chromosome DNA can be prepared for example according to a method of Smith et al. (Smith, M.G., Methods in Enzymology, Academic press, New York, 12, part A, p. 545 (1967)). The cloning of the lipase gene can be carried out according to Saiki et al.'s Polymerase Chain Reaction (PCR) process (Saiki, R.K. et al., Science, 230, 1350-1354, (1985)). At that time, the primer used for cloning the lipase gene should be selected from a well-preserved site or a site where it can be expected to have been well-preserved, by comparing and examining the amino acid sequences of known lipases. Further, when a DNA fragment containing a portion of the lipase gene obtained according to PCR method is used as a probe for cloning, it is possible to obtain a recombinant DNA containing a lipase gene from the chromosome DNA library of Pseudomonas cepacia IFO 14595 strain.

The region necessary for expressing the lipase gene can be determined by subcloning a recombinant DNA containing a lipase gene, obtained by screening, using various kinds of restriction enzymes, and observing the clear zone formability of the resulting transformed strain on a tributyrin-agar medium. The lipase gene and the DNA sequence of the region necessary for its expression can be determined according to a known method such as dideoxy method (Sanger. F. et al., Proc. Natl. Acad. Sci. U.S.A., 74, p. 5463 (1977)).

(2) Production of lipase into a cultured filtrate

When the thus obtained DNA fragment containing a lipase gene is introduced into a plasmid vector developed for producing a heterogeneous gene replicative inside Escherichia coli or Pseudomonas genus bacterium, it is possible to construct a recombinant DNA for lipase expression. As such a plasmid vector, pTV118N (made by Takara Shuzo Co., Ltd.), pKK 223-3 plasmid (made by Pharmacia Co., Ltd.), etc. for Escherichia coli expression, and pMMB66EH (Furste, J.P. et al., Gene, 48, p. 119-131 (1986)), etc. for Pseudomonas genus bacterium expression, are exemplified.

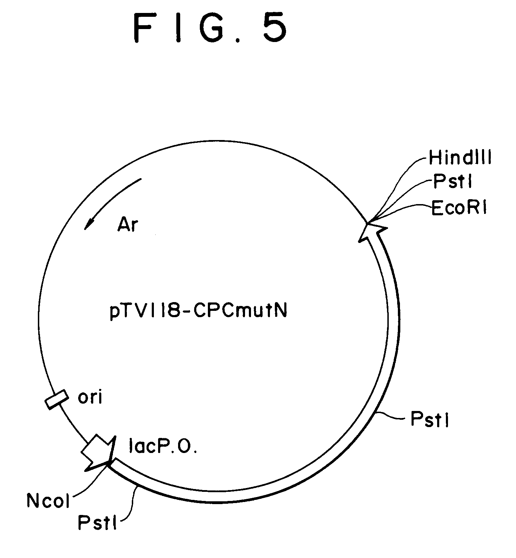

However, the secretory production of these lipases into a cultured filtrate are not expected from the above vectors. The secretory production of lipase into a cultured filtrate is achieved by constructing a system of a recombinant DNA and a host species bacterium, having various factors participating in the secretory production based upon these vectors, taken into consideration. For example, a recombinant DNA plasmid pTV-CPC mut N (shown in Fig. 5), constructed taking into consideration, a signal peptide of lipase and a gene adjusting lipase activity expression, is mentioned.

The present invention will be described in more detail by way of Example.

Example

(1) Preparation of chromosome DNA

Pseudomonas cepacia IFO 14595 strain was cultivated in an LB medium (1% bactotripton, 0.5% yeast extract and 0.5% NaCℓ) (200 mℓ) at 30°C overnight. The bacterial cells were harvested, followed by washing and suspending in a solution of 50 mM tris-HCℓ buffer (pH 8.0) (12 mℓ) and EDTA (10 mM). Lysozyme (1 mg) was added to the resulting suspension, followed by allowing the mixture to stand for one hour, adding to the resulting solution, protenase K (made by Merck Co., Ltd.) so as to give a concentration of 100 μg/mℓ, in the presence of 0.5% SDS, slowly shaking the mixture at 55°C for 3 hours for bacteriolysis of the bacterial cells, extracting the resulting sample with phenol, and precipitating with ethanol, to prepare a chromosome DNA.

(2) Cloning of lipase gene and analysis

As to the primer used in the lipase gene cloning according to PCR method, Pseudomonas fragi IFO 12049 strain lipase gene (Aoyama S. et al., FEBS Lett., 242, p. 36 (1988)), Pseudomonas sp. KWI-56 strain lipase gene (Iizumi T. et al., Agric. Biol. Chem., 55, p. 2349, (1991), Pseudomonas cepacia DSM 3959 strain lipase gene (Jørgensen S. et al., J. Bacteriol., 173, p. 559, (1991), etc. were compared and investigated, and DNA sequences corresponding to the following amino acids were selected:

| | | |
|---|---|---|
| | | Ala Ala Thr Arg Tyr Pro Ile |
| Sense chain primer AON-2 | 5'- | GCG GCG ACG CG(T/C) TA(C/T) CCG AT-3' |
| | | Trp Asn His Leu Asp Glu Ile |
| Antisense chain primer AOR-4 | 3'- | ACC TTG GT(A/G) GAG CTG CTC TAG-5' |

PCR method was carried out using a reagent kit solely used for PCR method (made by Takara Shuzo Co., Ltd.) and under the following conditions:

| Reaction solution | |
|---|---|
| Pseudomonas cepacia IFO14595 chromosome DNA | 1 μg |
| AON-2 primer | 100 pmol |
| AOR-4 primer | 100 pmol |
| dNTP solution | each, 1.0 mM |
| 10X reaction buffer | 10 μℓ |
| TaqDNA polymerase | 2.5 Unit |
| | Total 100 μℓ |

| Reaction conditions | |
|---|---|
| Thermal modification | 94 °C, one min. |
| Annealing | 62 °C, 1.5 min. |
| DNA synthesis | 72 °C, 1.5 min. |
| Cycle number | 30 times |

The thus amplified DNA fragment was recovered, followed by subcloning it into pUC 13 vector, and determining the DNA sequence according to dideoxy method, whereby it was confirmed that a part of the lipase gene was coded.

Next, using a DNA fragment obtained according to PCR method as a probe, Pseudomonas cepacia IFO 14595 strain chromosome DNA digested with a restriction enzyme, Eco RI, was subjected to Southern hybridization ( Southern, E.M., J.Mol. Biol., 98, p.5O3 ( 1975 )).

As a result, a DNA fragment complementary to the probe was detected at a position of about 13.5 kb. These methods were carried out according to a method of non-rdio system DNA marking and a detection kit (made by Beringer Mannheim Yamanouchi Co., Ltd.).

Next, Pseudomonas cepacia IFO 14595 strain chromosome DNA was digested with restriction enzyme Eco RI, followed by subjecting the resulting substance to electrophoresis in agarose gel, to recover its 13.5 kb fragment. This recovered DNA fragment was introduced into Eco RI site of Charomid 9-42 (made by Nippon Gene Co.,Ltd.) with a DNA ligase. The resulting DNA was infected with Escherichia coli VCS 257 strain, as a phage particle, using λ DNA in-vitro packaging kit Gigapack II PLUS (made by STRATAGEN Co., Ltd.).

This transformed strain was plated on an LB medium containing ampicillin (50 μg/mℓ). About 20,000 transformed strains obtained by cultivation at 37°C overnight were hybridized with the labeled probe obtained according to the above PCR method, followed by screening according to colony hybridization method .

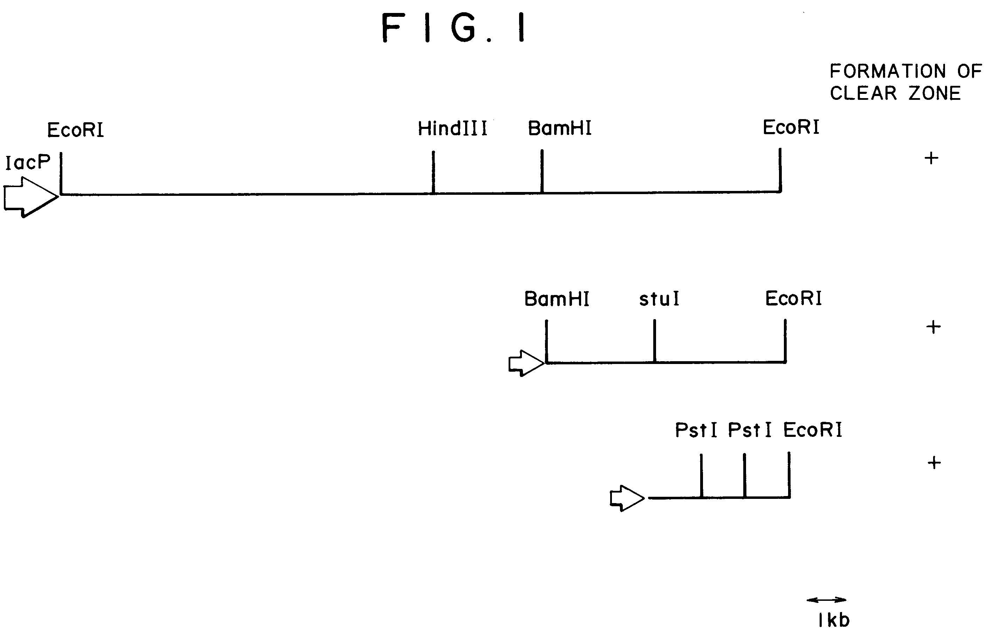

As a result, 50 positive clones could be obtained. With 7 positive clones among the above 50 positive clones, recombinant Charomid DNA was prepared, followed by analysis with a restriction enzeme. As a result, all the clones were same, and the recombinant Charomid DNA was named pCPC. Further, Escherichia coli having pCPC introduced therein had a clear zone-formability. Next, pCPC was digested with various kinds of restriction enzymes, followed by subcloning the respective DNA fragments in pUC 119 (made by Takara Shuzo Co., Ltd.), to transform Escherichia coli JM83 strain, and thereby investigate the presence or absence of clear zone formation on an LB medium containing tributyrin. Fig. 1 shows the results of a defect experiment, wherein a restriction enzyme map of pCPC and clear zone formation were employed as indications. It has been suggested that a lipase-coding gene and a region necessary for expressing the gene are present in StuI-EcoRI 2.8 kb DNA fragment. This plasmid having the StuI-EcoRI 2.8 kb DNA fragment was named pCPC-SE.

(3) Determination of nucleotide sequence

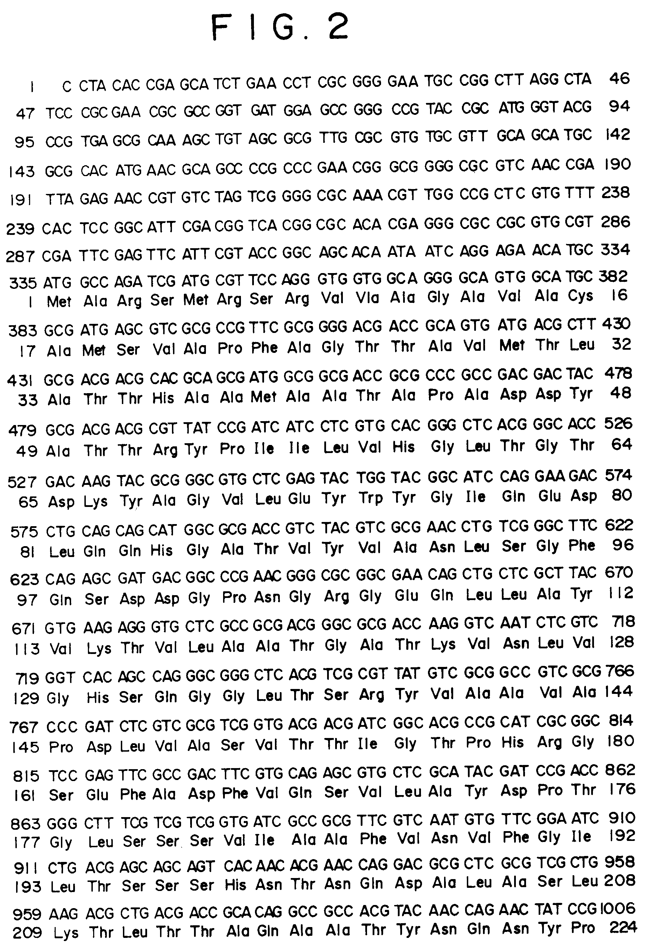

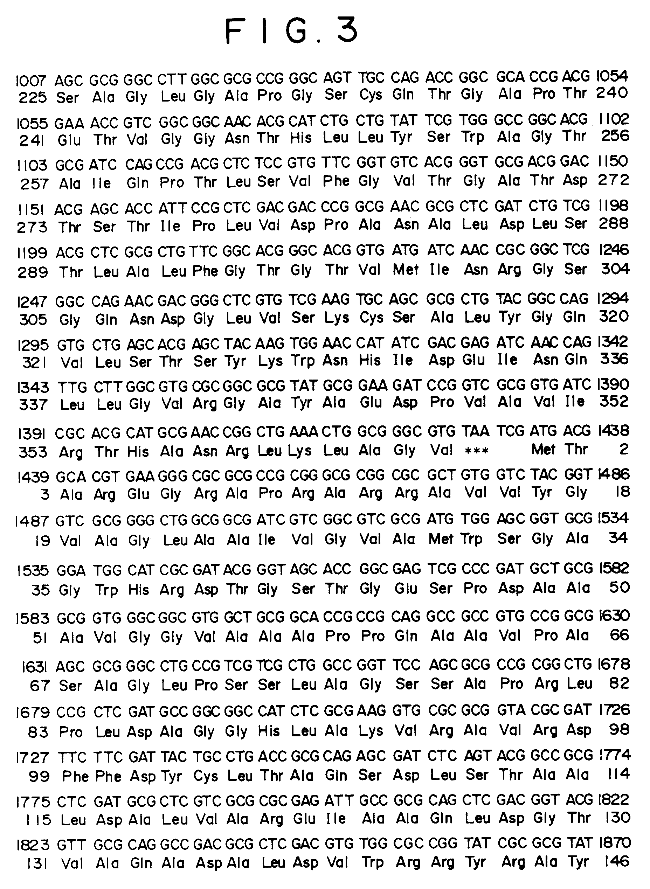

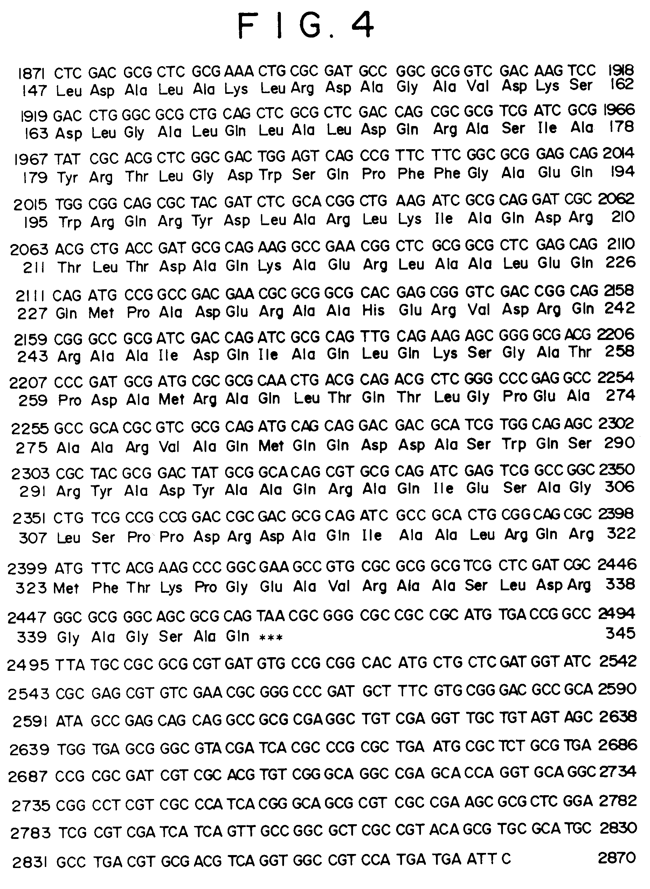

The nucleotide sequence of pCPC-SE-inserted DNA fragment was determined according to dideoxy method. As a result, an open reading frame coding the lipase gene of sequence No. 1 and an open reading frame coding factor necessary for expressing the lipase activity of sequence No. 2, downstream of the above frame, were confirmed to be present. Further, it was found that a signal peptide was present in the open reading frame of sequence No. 1, from comparison with amino acids of lipase of Japanese patent application laid-open Nos. Hei 3-87187 and Hei 3-47079. The DNA sequence of the insert DNA fragment and the amino acid sequences of the corresponding lipase and the gene adjusting the expression of activity of lipase are shown in Figs. 2, 3 and 4 (Note: one gene as a whole).

(4) Secretory production of lipase into cultured filtrate:

Using a variant-introducing primer of

5' -AGGAGAACATCCATGGCCAGA-3'
*

(*: variant-introduced part),

a restriction enzyme NcoI site was introduced into the initiation codon part of the lipase gene in the pCPC-SE, to prepare pCPC-SE mut N. The introduction of the variant was carried out according to a procedure method of Mutan-K made by Takara Shuzo Co., Ltd. NcoI-EcoRI 2.5 kb DNA fragment of pCPC-SE mut N was introduced into NcoI-EcoRI site of pTV118N (made by Takarashuzo Co., Ltd.), to construct pTV118-CPC mut N of lipase expression (see Fig. 5). This pTV118-CPC mut N was introduced into Escherichia coli JM 109 strain. This Escherichia coli JM 109 (pTV118-CPC mut N) strain was cultivated on LB medium at 37°C overnight, followed by planting its 200 μℓ on a new LB medium.

After cultivation at 37°C for 2 hours, IPTG (Isopropyl β-D-thiogalactopyranoside) was added so as to give a final concentration of 1 mM, followed by cultivating the resulting culture for two hours under the same conditions, and measuring the lipase activity of the cultured filtrate using Lipase kit S (made by Dainippon Seiyaku Co., Ltd.). As a result, the activity was 1,400 BALB unit per 100 μℓ of the cultured filtrate

The value of Escherichia coli JM 109 strain measured under the same conditions was lower than the lower limit of measurement sensitivity (50 BALB unit). In addition, the measurement method and the calculation method of measurement unit were carried out according to lipase kit S method.

The preparation of plasmid DNA from the bacteria, the reaction using restriction enzymes, etc. and the treatment of DNA fragment in the above Example were carried out according to usually employed methods, unless otherwise indicated. Further, as to the restriction enzyme, DNA ligase, etc., those made by Takara Shuzo Co., Ltd., and Nippon Gene Co., Ltd. were used.

As described above, according to the present invention, a gene coding a lipase is provided, and when a transformed strain having the gene incorporated therein is cultivated, it is possible to carry out secretory production of lipase into the cultured filtrate. The thus produced lipase can be used in the field of commercial mass production for cheap supply, etc.

## Claims

**1.** A lipase gene originated from Pseudomonas genus bacterium, characterized by having a DNA sequence of sequence No. 1:

| | |
|---|---|
| Length of sequence: | 1095 |
| Type of sequence: | nucleic acid |
| Number of chains: | double-stranded |
| Topology: | linear |
| Kind of sequence: | Genomic DNA |
| Origin: | |
| | Name of organism: Pseudomonas cepacia |
| | Name of strain: IFO 14595 |
| Sequence: | |

```
ATGGCCAGAT CGATGCGTTC CAGGGTGGTG GCAGGGGCAG TGGCATGCGC GATGAGCGTC    60
GCGCCGTTCG CGGGGACGAC CGCAGTGATG ACGCTTGCGA CGACGCACGC AGCGATGGCG   120
GCGACCGCGC CCGCCGACGA CTACGCGACG ACGCGTTATC CGATCATCCT CGTGCACGGG   180
CTCACGGGCA CCGACAAGTA CGCGGGCGTG CTCGAGTACT GGTACGGCAT CCAGGAAGAC   240
CTGCAGCAGC ATGGCGCGAC CGTCTACGTC GCGAACCTGT CGGGCTTCCA GAGCGATGAC   300
GGCCCGAACG GGCGCGGCGA ACAGCTGCTC GCTTACGTGA AGACGGTGCT CGCCGCGACG   360
GGCGCGACCA AGGTCAATCT CGTCGGTCAC AGCCAGGGCG GGCTCACGTC GCGTTATGTC   420
GCGGCCGTCG CGCCCGATCT CGTCGCGTCG GTGACGACGA TCGGCACGCC GCATCGCGGC   480
TCCGAGTTCG CCGACTTCGT GCAGAGCGTG CTCGCATACG ATCCGACCGG GCTTTCGTCG   540
TCGGTGATCG CCGCGTTCGT CAATGTGTTC GGAATCCTGA CGAGCAGCAG TCACAACACG   600
AACCAGGACG CGCTCGCGTC GCTGAAGACG CTGACGACCG CACAGGCCGC CACGTACAAC   660
CAGAACTATC CGAGCGCGGG CCTTGGCGCG CCGGGCAGTT GCCAGACCGG CGCACCGACG   720
GAAACCGTCG GCGGCAACAC GCATCTGCTG TATTCGTGGG CCGGCACGGC GATCCAGCCG   780
ACGCTCTCCG TGTTCGGTGT CACGGGTGCG ACGGACACGA GCACCATTCC GCTCGTCGAC   840
CCGGCGAACG CGCTCGATCT GTCGACGCTC GCGCTGTTCG GCACGGGCAC GGTGATGATC   900
AACCGCGGCT CGGGCCAGAA CGACGGGCTC GTGTCGAAGT GCAGCGCGCT GTACGGCCAG   960
GTGCTGAGCA CGAGCTACAA GTGGAACCAT ATCGACGAGA TCAACCAGTT GCTTGGCGTG  1020
CGCGGCGCGT ATGCGGAAGA TCCGGTCGCG GTGATCCGCA CGCATGCGAA CCGGCTGAAA  1080
CTGGCGGGCG TGTAA                                                   1095
```

**2.** A gene coding a factor necessary for expressing the lipase activity originated from Pseudomonas genus bacterium, characterized by having a DNA sequence of sequence No. 2:

Length of sequence: 1035
Type of sequence: nucleic acid
Number of chains: double-stranded
Topology: linear
Kind of sequence: Genomic DNA
Origin:
Name of organism: Pseudomonas cepacia
Name of strain: IFO 14595
Sequence:

```
ATGACGGCAC GTGAAGGGCG CGCGCCGCGG GCGCGGCGCG CTGTGGTCTA CGGTGTCGCG   60
GGGCTGGCGG CGATCGTCGG CGTCGCGATG TGGAGCGGTG CGGGATGGCA TCGCGATACG  120
GGTAGCACCG GCGAGTCGCC CGATGCTGCG GCGGTGGGCG GCGTGGCTGC GGCACCGCCG  180
CAGGCCGCCG TGCCGGCGAG CGCGGGCCTG CCGTCGTCGC TGGCCGGTTC CAGCGCGCCG  240
CGGCTGCCGC TCGATGCCGG CGGCCATCTC GCGAAGGTGC GCGCGGTACG CGATTTCTTC  300
GATTACTGCC TGACCGCGCA GAGCGATCTC AGTACGGCCG CGCTCGATGC GCTCGTCGCG  360
CGCGAGATTG CCGCGCAGCT CGACGGTACG GTTGCGCAGG CCGACGCGCT CGACGTGTGG  420
CGCCGGTATC GCGCGTATCT CGACGCGCTC GCGAAACTGC GCGATGCCGG CGCGGTCGAC  480
AAGTCCGACC TGGGCGCGCT GCAGCTCGCG CTCGACCAGC GCGCGTCGAT CGCGTATCGC  540
ACGCTCGGCG ACTGGAGTCA GCCGTTCTTC GGCGCGGAGC AGTGGCGGCA GCGCTACGAT  600
CTCGCACGGC TGAAGATCGC GCAGGATCGC ACGCTGACCG ATGCGCAGAA GGCCGAACGG  660
CTCGCGGCGC TCGAGCAGCA GATGCCGGCC GACGAACGCG CGGCGCACGA GCGGGTCGAC  720
CGGCAGCGGG CCGCGATCGA CCAGATCGCG CAGTTGCAGA AGAGCGGGGC GACGCCCGAT  780
GCGATGCGCG CGCAACTGAC GCAGACGCTC GGGCCCGAGG CCGCCGCACG CGTCGCGCAG  840
ATGCAGCAGG ACGACGCATC GTGGCAGAGC CGCTACGCGG ACTATGCGGC ACAGCGTGCG  900
CAGATCGAGT CGGCCGGCCT GTCGCCGCCG GACCGCGACG CGCAGATCGC CGCACTGCGG  960
CAGCGCATGT TCACGAAGCC CGGCGAAGCC GTGCGCGCGG CGTCGCTCGA TCGCGGCGCG 1020
GGCAGCGCGC AGTAA                                                  1035
```

**3.** A lipase gene according to claim 1 , wherein said Pseudomonas genus bacterium is Pseudomonas cepacia IFO 14595 strain.

**4.** A recombinant DNA having incorporated therein, a lipase gene or a gene coding a factor necessary for expressing a lipase gene according to either one of claims 1 , 2 or 3 .

**5.** A host species bacterium transformed by said recombinant DNA according to claim 4 .

**6.** A process for producing a lipase, which comprises cultivating said host species bacterium according to claim 5 and secretorily producing a lipase in the cultured filtrate.

**7.** A process for producing a lipase according to claim 6 wherein the vector according to claim 4 is a vector for Eschericia coli and the host genus bacterium according to claim 5 is Escherichia coli.

FIG. 1

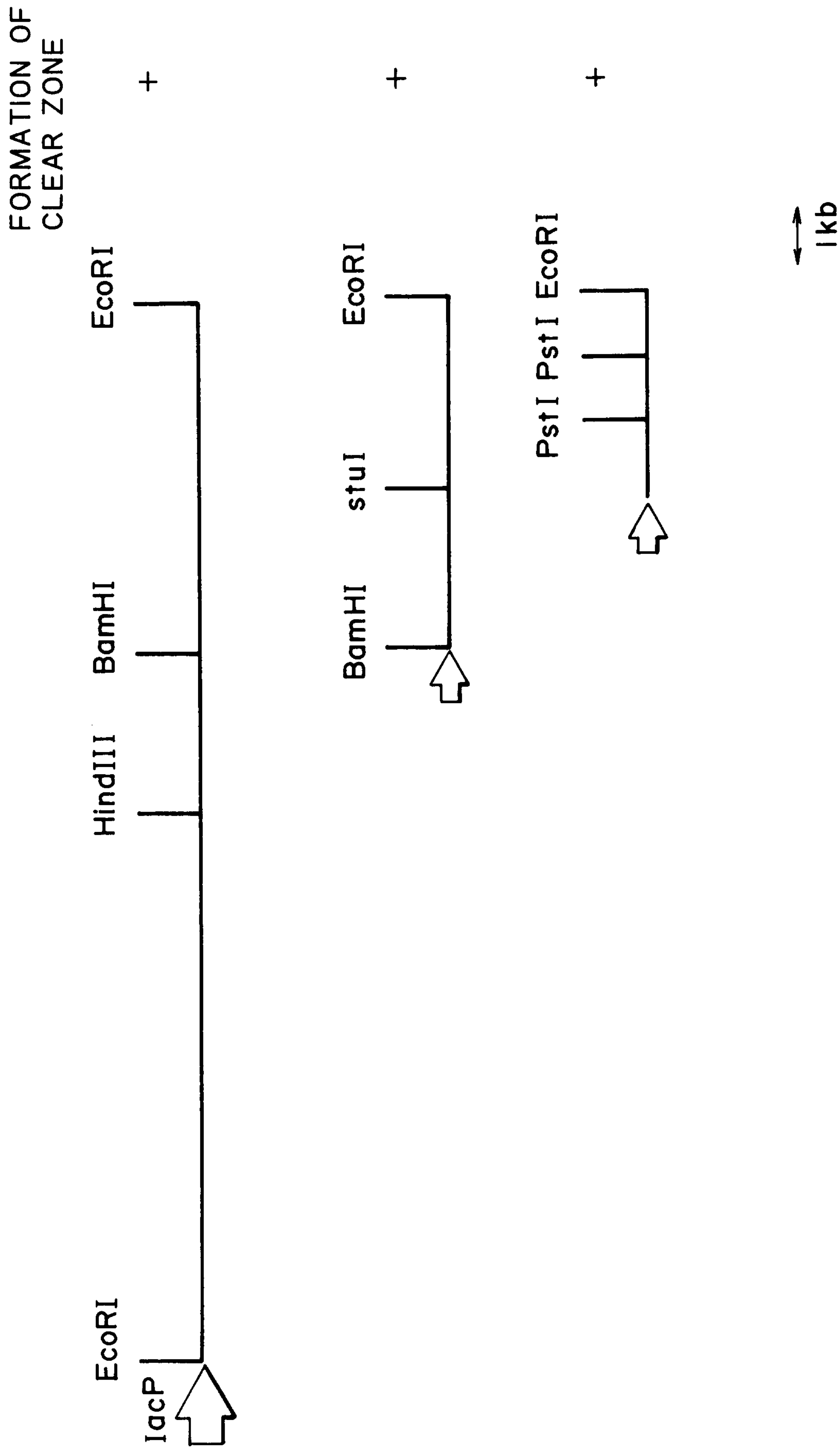
FORMATION OF CLEAR ZONE
+
+
+
EcoRI
BamHI
HindIII
EcoRI
lacP
EcoRI
stuI
BamHI
PstI PstI EcoRI
1kb

# FIG. 2

1 C CTA CAC CGA GCA TCT GAA CCT CGC GGG GAA TGC CGG CTT AGG CTA 46

47 TCC CGC GAA CGC GCC GGT GAT GGA GCC GGG CCG TAC CGC ATG GGT ACG 94

95 CCG TGA GCG CAA AGC TGT AGC GCG TTG CGC GTG TGC GTT GCA GCA TGC 142

143 GCG CAC ATG AAC GCA GCC CCG CCC GAA CGG GCG GGG CGC GTC AAC CGA 190

191 TTA GAG AAC CGT GTC TAG TCG GGG CGC AAA CGT TGG CCG CTC GTG TTT 238

239 CAC TCC GGC ATT CGA CGG TCA CGG CGC ACA CGA GGG CGC CGC GTG CGT 286

287 CGA TTC GAG TTC ATT CGT ACC GGC AGC ACA ATA ATC AGG AGA ACA TGC 334

335 ATG GCC AGA TCG ATG CGT TCC AGG GTG GTG GCA GGG GCA GTG GCA TGC 382

1 Met Ala Arg Ser Met Arg Ser Arg Val Vla Ala Gly Ala Val Ala Cys 16

383 GCG ATG AGC GTC GCG CCG TTC GCG GGG ACG ACC GCA GTG ATG ACG CTT 430

17 Ala Met Ser Val Ala Pro Phe Ala Gly Thr Thr Ala Val Met Thr Leu 32

431 GCG ACG ACG CAC GCA GCG ATG GCG GCG ACC GCG CCC GCC GAC GAC TAC 478

33 Ala Thr Thr His Ala Ala Met Ala Ala Thr Ala Pro Ala Asp Asp Tyr 48

479 GCG ACG ACG CGT TAT CCG ATC ATC CTC GTG CAC GGG CTC ACG GGC ACC 526

49 Ala Thr Thr Arg Tyr Pro Ile Ile Leu Val His Gly Leu Thr Gly Thr 64

527 GAC AAG TAC GCG GGC GTG CTC GAG TAC TGG TAC GGC ATC CAG GAA GAC 574

65 Asp Lys Tyr Ala Gly Val Leu Glu Tyr Trp Tyr Gly Ile Gln Glu Asp 80

575 CTG CAG CAG CAT GGC GCG ACC GTC TAC GTC GCG AAC CTG TCG GGC TTC 622

81 Leu Gln Gln His Gly Ala Thr Val Tyr Val Ala Asn Leu Ser Gly Phe 96

623 CAG AGC GAT GAC GGC CCG AAC GGG CGC GGC GAA CAG CTG CTC GCT TAC 670

97 Gln Ser Asp Asp Gly Pro Asn Gly Arg Gly Glu Gln Leu Leu Ala Tyr 112

671 GTG AAG AGG GTG CTC GCC GCG ACG GGC GCG ACC AAG GTC AAT CTC GTC 718

113 Val Lys Thr Val Leu Ala Ala Thr Gly Ala Thr Lys Val Asn Leu Val 128

719 GGT CAC AGC CAG GGC GGG CTC ACG TCG CGT TAT GTC GCG GCC GTC GCG 766

129 Gly His Ser Gln Gly Gly Leu Thr Ser Arg Tyr Val Ala Ala Val Ala 144

767 CCC GAT CTC GTC GCG TCG GTG ACG ACG ATC GGC ACG CCG CAT CGC GGC 814

145 Pro Asp Leu Val Ala Ser Val Thr Thr Ile Gly Thr Pro His Arg Gly 180

815 TCC GAG TTC GCC GAC TTC GTG CAG AGC GTG CTC GCA TAC GAT CCG ACC 862

161 Ser Glu Phe Ala Asp Phe Val Gln Ser Val Leu Ala Tyr Asp Pro Thr 176

863 GGG CTT TCG TCG TCG GTG ATC GCC GCG TTC GTC AAT GTG TTC GGA ATC 910

177 Gly Leu Ser Ser Ser Val Ile Ala Ala Phe Val Asn Val Phe Gly Ile 192

911 CTG ACG AGC AGC AGT CAC AAC ACG AAC CAG GAC GCG CTC GCG TCG CTG 958

193 Leu Thr Ser Ser Ser His Asn Thr Asn Gln Asp Ala Leu Ala Ser Leu 208

959 AAG ACG CTG ACG ACC GCA CAG GCC GCC ACG TAC AAC CAG AAC TAT CCG 1006

209 Lys Thr Leu Thr Thr Ala Gln Ala Ala Thr Tyr Asn Gln Asn Tyr Pro 224

# F I G. 3

1007 AGC GCG GGC CTT GGC GCG CCG GGC AGT TGC CAG ACC GGC GCA CCG ACG 1054

225 Ser Ala Gly Leu Gly Ala Pro Gly Ser Cys Gln Thr Gly Ala Pro Thr 240

1055 GAA ACC GTC GGC GGC AAC ACG CAT CTG CTG TAT TCG TGG GCC GGC ACG 1102

241 Glu Thr Val Gly Gly Asn Thr His Leu Leu Tyr Ser Trp Ala Gly Thr 256

1103 GCG ATC CAG CCG ACG CTC TCC GTG TTC GGT GTC ACG GGT GCG ACG GAC 1150

257 Ala Ile Gln Pro Thr Leu Ser Val Phe Gly Val Thr Gly Ala Thr Asp 272

1151 ACG AGC ACC ATT CCG CTC GAC GAC CCG GCG AAC GCG CTC GAT CTG TCG 1198

273 Thr Ser Thr Ile Pro Leu Val Asp Pro Ala Asn Ala Leu Asp Leu Ser 288

1199 ACG CTC GCG CTG TTC GGC ACG GGC ACG GTG ATG ATC AAC CGC GGC TCG 1246

289 Thr Leu Ala Leu Phe Gly Thr Gly Thr Val Met Ile Asn Arg Gly Ser 304

1247 GGC CAG AAC GAC GGG CTC GTG TCG AAG TGC AGC GCG CTG TAC GGC CAG 1294

305 Gly Gln Asn Asp Gly Leu Val Ser Lys Cys Ser Ala Leu Tyr Gly Gln 320

1295 GTG CTG AGC ACG AGC TAC AAG TGG AAC CAT ATC GAC GAG ATC AAC CAG 1342

321 Val Leu Ser Thr Ser Tyr Lys Trp Asn His Ile Asp Glu Ile Asn Gln 336

1343 TTG CTT GGC GTG CGC GGC GCG TAT GCG GAA GAT CCG GTC GCG GTG ATC 1390

337 Leu Leu Gly Val Arg Gly Ala Tyr Ala Glu Asp Pro Val Ala Val Ile 352

1391 CGC ACG CAT GCG AAC CGG CTG AAA CTG GCG GGC GTG TAA TCG ATG ACG 1438

353 Arg Thr His Ala Asn Arg Leu Lys Leu Ala Gly Val *** Met Thr 2

1439 GCA CGT GAA GGG CGC GCG CCG CGG GCG CGG CGC GCT GTG GTC TAC GGT 1486

3 Ala Arg Glu Gly Arg Ala Pro Arg Ala Arg Arg Ala Val Val Tyr Gly 18

1487 GTC GCG GGG CTG GCG GCG ATC GTC GGC GTC GCG ATG TGG AGC GGT GCG 1534

19 Val Ala Gly Leu Ala Ala Ile Val Gly Val Ala Met Trp Ser Gly Ala 34

1535 GGA TGG CAT CGC GAT ACG GGT AGC ACC GGC GAG TCG CCC GAT GCT GCG 1582

35 Gly Trp His Arg Asp Thr Gly Ser Thr Gly Glu Ser Pro Asp Ala Ala 50

1583 GCG GTG GGC GGC GTG GCT GCG GCA CCG CCG CAG GCC GCC GTG CCG GCG 1630

51 Ala Val Gly Gly Val Ala Ala Ala Pro Pro Gln Ala Ala Val Pro Ala 66

1631 AGC GCG GGC CTG CCG TCG TCG CTG GCC GGT TCC AGC GCG CCG CGG CTG 1678

67 Ser Ala Gly Leu Pro Ser Ser Leu Ala Gly Ser Ser Ala Pro Arg Leu 82

1679 CCG CTC GAT GCC GGC GGC CAT CTC GCG AAG GTG CGC GCG GTA CGC GAT 1726

83 Pro Leu Asp Ala Gly Gly His Leu Ala Lys Val Arg Ala Val Arg Asp 98

1727 TTC TTC GAT TAC TGC CTG ACC GCG CAG AGC GAT CTC AGT ACG GCC GCG 1774

99 Phe Phe Asp Tyr Cys Leu Thr Ala Gln Ser Asp Leu Ser Thr Ala Ala 114

1775 CTC GAT GCG CTC GTC GCG CGC GAG ATT GCC GCG CAG CTC GAC GGT ACG 1822

115 Leu Asp Ala Leu Val Ala Arg Glu Ile Ala Ala Gln Leu Asp Gly Thr 130

1823 GTT GCG CAG GCC GAC GCG CTC GAC GTG TGG CGC CGG TAT CGC GCG TAT 1870

131 Val Ala Gln Ala Asp Ala Leu Asp Val Trp Arg Arg Tyr Arg Ala Tyr 146

# FIG. 4

1871 CTC GAC GCG CTC GCG AAA CTG CGC GAT GCC GGC GCG GTC GAC AAG TCC 1918
147 Leu Asp Ala Leu Ala Lys Leu Arg Asp Ala Gly Ala Val Asp Lys Ser 162

1919 GAC CTG GGC GCG CTG CAG CTC GCG CTC GAC CAG CGC GCG TCG ATC GCG 1966
163 Asp Leu Gly Ala Leu Gln Leu Ala Leu Asp Gln Arg Ala Ser Ile Ala 178

1967 TAT CGC ACG CTC GGC GAC TGG AGT CAG CCG TTC TTC GGC GCG GAG CAG 2014
179 Tyr Arg Thr Leu Gly Asp Trp Ser Gln Pro Phe Phe Gly Ala Glu Gln 194

2015 TGG CGG CAG CGC TAC GAT CTC GCA CGG CTG AAG ATC GCG CAG GAT CGC 2062
195 Trp Arg Gln Arg Tyr Asp Leu Ala Arg Leu Lys Ile Ala Gln Asp Arg 210

2063 ACG CTG ACC GAT GCG CAG AAG GCC GAA CGG CTC GCG GCG CTC GAG CAG 2110
211 Thr Leu Thr Asp Ala Gln Lys Ala Glu Arg Leu Ala Ala Leu Glu Gln 226

2111 CAG ATG CCG GCC GAC GAA CGC GCG GCG CAC GAG CGG GTC GAC CGG CAG 2158
227 Gln Met Pro Ala Asp Glu Arg Ala Ala His Glu Arg Val Asp Arg Gln 242

2159 CGG GCC GCG ATC GAC CAG ATC GCG CAG TTG CAG AAG AGC GGG GCG ACG 2206
243 Arg Ala Ala Ile Asp Gln Ile Ala Gln Leu Gln Lys Ser Gly Ala Thr 258

2207 CCC GAT GCG ATG CGC GCG CAA CTG ACG CAG ACG CTC GGG CCC GAG GCC 2254
259 Pro Asp Ala Met Arg Ala Gln Leu Thr Gln Thr Leu Gly Pro Glu Ala 274

2255 GCC GCA CGC GTC GCG CAG ATG CAG CAG GAC GAC GCA TCG TGG CAG AGC 2302
275 Ala Ala Arg Val Ala Gln Met Gln Gln Asp Asp Ala Ser Trp Gln Ser 290

2303 CGC TAC GCG GAC TAT GCG GCA CAG CGT GCG CAG ATC GAG TCG GCC GGC 2350
291 Arg Tyr Ala Asp Tyr Ala Ala Gln Arg Ala Gln Ile Glu Ser Ala Gly 306

2351 CTG TCG CCG CCG GAC CGC GAC GCG CAG ATC GCC GCA CTG CGG CAG CGC 2398
307 Leu Ser Pro Pro Asp Arg Asp Ala Gln Ile Ala Ala Leu Arg Gln Arg 322

2399 ATG TTC ACG AAG CCC GGC GAA GCC GTG CGC GCG GCG TCG CTC GAT CGC 2446
323 Met Phe Thr Lys Pro Gly Glu Ala Val Arg Ala Ala Ser Leu Asp Arg 338

2447 GGC GCG GGC AGC GCG CAG TAA CGC GGG CGC CGC CGC ATG TGA CCG GCC 2494
339 Gly Ala Gly Ser Ala Gln *** 345

2495 TTA TGC CGC GCG CGT GAT GTG CCG CGG CAC ATG CTG CTC GAT GGT ATC 2542

2543 CGC GAG CGT GTC GAA CGC GGG CCC GAT GCT TTC GTG CGG GAC GCC GCA 2590

2591 ATA GCC GAG CAG CAG GCC GCG CGA GGC TGT CGA GGT TGC TGT AGT AGC 2638

2639 TGG TGA GCG GGC GTA CGA TCA CGC CCG CGC TGA ATG CGC TCT GCG TGA 2686

2687 CCG CGC GAT CGT CGC ACG TGT CGG GCA GGC CGA GCA CCA GGT GCA GGC 2734

2735 CGG CCT CGT CGC CCA TCA CGG GCA GCG CGT CGC CGA AGC GCG CTC GGA 2782

2783 TCG CGT CGA TCA TCA GTT GCC GGC GCT CGC CGT ACA GCG TGC GCA TGC 2830

2831 GCC TGA CGT GCG ACG TCA GGT GGC CGT CCA TGA TGA ATT C 2870

# F I G. 5

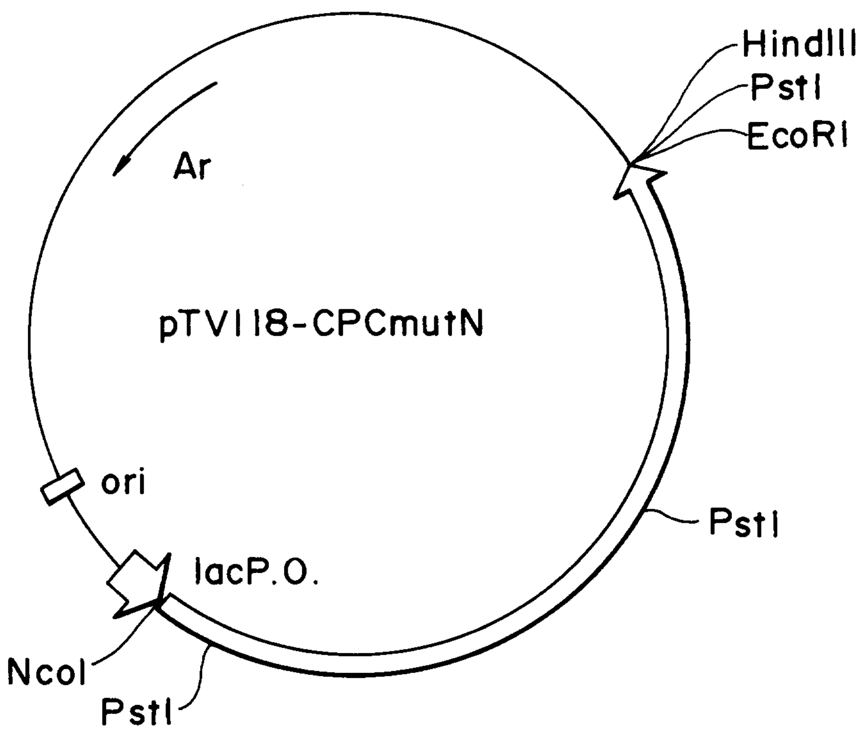
HindIII
PstI
EcoRI
Ar
pTV118-CPCmutN
ori
lacP.O.
PstI
NcoI
PstI